# EUROPÄISCHE PATENTSCHRIFT

(11) **EP 3 465 128 B1**
(45) Veröffentlichungstag und Bekanntmachung des Hinweises auf die Patenterteilung: **05.08.2020**
(21) Anmeldenummer: 17717699.7
(22) Anmeldetag: 13.04.2017
(51) Int. Cl.: G01M 15/10, G01N 1/22, H05K 5/00

(54) **SCHALTSCHRANK FÜR ABGASMESSANLAGEN**
SWITCH CABINET FOR EXHAUST-GAS MEASUREMENT INSTALLATIONS
ARMOIRE DE COMMANDE POUR SYSTÈMES DE MESURE DE GAZ D'ÉCHAPPEMENT

(30) Priorität: 31.05.2016 DE 102016110063
(43) Veröffentlichungstag der Anmeldung: 10.04.2019
(73) Patentinhaber: AVL Emission Test Systems GmbH, 41460 Neuss (DE)
(72) Erfinder: DICKOW, Achim, 42555 Velbert (DE)
(74) Vertreter: terpatent Patentanwälte ter Smitten Eberlein-Van Hoof Rütten Daubert
(86) Internationale Anmeldenummer: PCT/EP2017/058929
(87) Internationale Veröffentlichungsnummer: WO 2017/207159

(56) Entgegenhaltungen:
- Siemens AG: "Analytical Application Sets - Continuous emission monitoring", , 2014, XP055245324, Gefunden im Internet: URL:http://www.automation.siemens.com/sc-s tatic/catalogs/catalog/pa/PA01/en/PA01_en_ Kap04.pdf [gefunden am 2016-01-27]
- Rosemount Analytical: "GMP 1000M CEMS Product Data Sheet GMP 1000M Continuous Emissions Monitoring System (CEMS) with MLT Analyzer", , Juli 2013 (2013-07), XP055380522, Gefunden im Internet: URL:http://www.emerson.com/documents/autom ation/69450.pdf [gefunden am 2017-06-12]
- Sick AG: "PowerCEMS50 - EFFICIENT CEMS SYSTEM FOR POWER PLANTS", , April 2015 (2015-04), Seiten 1-8, XP055380734, Gefunden im Internet: URL:https://www.sick.com/media/docs/6/86/1 86/Product_information_PowerCEMS50_Efficie nt_Cems_system_for_power_plants_en_IM00551 86.PDF [gefunden am 2017-06-12]

## Beschreibung

Die Erfindung betrifft Schaltschrank für Abgasmessanlagen mit einem Schrankkörper, der sich aus vier seitlich begrenzenden Wänden, von denen eine als Decke, eine als Boden und zwei als Seitenwände dienen, sowie einer Rückwand zusammensetzt und durch eine an der Frontseite angeordnete Tür verschließbar ist, wobei im Schrankkörper Messgeräte und Gasanschlüsse angeordnet sind, wobei sich die Gasanschlüsse durch die Wände des Schrankkörpers erstrecken und jeweils mit mindestens einem innerhalb des Schrankkörpers angeordneten Ventil zusammenwirken.

Derartige Schaltschränke werden insbesondere in der Automobilindustrie zur Bestimmung des Emissionsverhaltens von Kraftfahrzeugen verwendet. Das Kraftfahrzeug durchfahrt auf einem Rollenprüfstand einen definierten Fahrzyklus, wobei die dabei emittierten Abgase gesammelt, gegebenenfalls verdünnt und einem als Analyseeinheit ausgeführten Schaltschrank zugeführt werden. Im Schaltschrank sind neben unterschiedlichen Messgeräten zur Bestimmung verschiedener Bestandteile des Abgases, wie beispielsweise Kohlenstoffdioxid, Kohlenwasserstoff, oder Stickoxide, auch eine Stromversorgungseinheit, eine Ansteuereinheit, die Elektronik sowie Kühleinheiten angeordnet. Entsprechend weist der Schaltschrank eine Vielzahl an Anschlüssen und Leitungen auf, über die Messgas, Nullgas und Kalibriergas zu den entsprechenden Einheiten geführt werden müssen. Außerdem werden derartige Schaltschränke als Gasverteilerschränke eingesetzt, die im Wesentlichen Druckregler und Drucksensoren beinhalten, über die der Systemdruck auf den Anlagendruck verändert wird.

Ein Schaltschrank für eine Abgasmessanlage ist beispielsweise aus der EP 2 317 833 A1 bekannt. Dieser Schaltschrank besteht aus zwei Schrankteilen. Im vorderen Schrankteil sind Messgeräte sowie ein Rechner und im hinteren Schrankteil eine Einlassschnittstelle zum Einleiten von Messgasen, Messgaspumpen, Ventilen und Druckminderer angeordnet. Die Einlassschnittstelle ist in der Decke des hinteren Schrankteils ausgebildet und weist viele nebeneinander angeordnete Einlassleitungen auf, die im hinteren Abschnitt des Schaltschranks senkrecht verlaufen. Die Messgaspumpen, Ventile und Druckminderer sind, von der offenen Frontseite des hinteren Schaltschranks betrachtet, vor den Einlassleitungen angeordnet.

Problematisch an der in der EP 2 317 833 A1 beschriebenen Ausführung und grundsätzlich bei derartigen Schaltschränken ist, dass die Schaltschränke kompakt und bauraumsparend ausgeführt sein sollen, wodurch jedoch die im Schaltschrank angeordneten Komponenten, wie beispielsweise die Messgeräte, die Ventile und die Gasanschlüsse nicht ausreichend gut zugänglich sind, um insbesondere die Wartung des Schaltschranks zu erleichtern.

Weitere bekannte Abgasmessanlagen in Schaltschränken sind Set CEM von Siemens und GMP 1000M von Rosemount Analytical.

Es stellt sich daher die Aufgabe, einen Schaltschrank für Abgasmessanlagen derart weiterzuentwickeln, dass der Schaltschrank bauraumsparend ausgeführt ist und dennoch die im Schaltschrank angeordneten Komponenten, insbesondere die Ventile und die Gasanschlüsse, leicht zugänglich sind.

Diese Aufgabe wird durch einen Schaltschrank für Abgasmessanlagen mit den Merkmalen des Hauptanspruchs 1 gelöst.

Dadurch, dass mindestens ein erster Gasanschluss in einer ersten Horizontalebene und einer ersten Vertikalebene angeordnet ist und mindestens ein zweiter Gasanschluss in einer zur ersten Horizontalebene versetzten zweiten Horizontalebene und einer zur ersten Vertikalebene versetzten zweiten Vertikalebene angeordnet ist, wobei die Decke oder eine der Seitenwände stufenförmig mit einer ersten Stufe und einer zweiten Stufe ausgeführt sind, wobei an der ersten Stufe der mindestens eine erste Gasanschluss und an der zweiten Stufe der mindestens eine zweite Gasanschluss angeordnet sind, können die Gasanschlüsse und die Ventile in dem Schaltschrank derart angeordnet werden, dass die Gasanschlüsse auf eine einfache Weise von außen und die Ventile durch die offene Frontseite zugänglich sind. Durch die horizontal und vertikal versetzte Anordnung des zweiten Gasanschlusses relativ zum ersten Gasanschluss müssen keine Leitungen oder andere Elemente demontiert werden, um auf die Ventile und die Gasanschlüsse der jeweils anderen Ebene zugreifen zu können. Außerdem kann durch die zueinander versetzte Anordnung des ersten Gasanschluss und des zweiten Gasanschlusses der Bauraum des Schaltschranks reduziert werden. Durch die stufenförmige Ausgestaltung der Decke oder der Seitenwände erfolgt auf eine einfache und kostengünstige Weise die zueinander horizontale und vertikal versetzte Anordnung der Gasanschlüsse,

Vorzugsweise ist der mindestens eine erste Gasanschluss näher zur Tür angeordnet als der mindestens eine zweite Gasanschluss.

Vorzugsweise sind der mindestens eine erste Gasanschluss und der mindestens eine zweite Gasanschluss vertikal ausgerichtet und erstrecken sich durch die Decke des Schrankkörpers, wobei der mindestens eine erste Gasanschluss von der mittleren Horizontalebene des Schrankkörpers weiter außen angeordnet ist als der mindestens eine zweite Gasanschluss. Alternativ sind der mindestens eine erste Gasanschluss und der mindestens eine zweite Gasanschluss horizontal ausgerichtet und erstrecken sich durch eine Seitenwand des Schrankkörpers, wobei der mindestens eine erste Gasanschluss von der mittleren Vertikalebene des Schrankkörpers weiter außen angeordnet ist als der mindestens eine zweite Gasanschluss. Durch eine derartige Anordnung der Gasanschlüsse sind die Ventile der Gasanschlüsse, insbesondere die Ventile, die mit dem zweiten Gasanschluss zusammenwirken, durch die offene Frontseite leicht zugänglich, da keine Leitungen vor der zweiten Anschlussleiste entlang geführt werden müssen.

In einer bevorzugten Ausgestaltung ist zwischen den beiden Stufen ein entfernbares Deckenelement angeordnet ist. Durch das entfernbare Deckenelement ist der Schaltschrank zur Umgebung abgeschlossen und ermöglicht durch ein einfaches Entfernen des Deckenelements eine bessere Zugänglichkeit des zweiten Gasanschlusses.

Vorzugsweise sind der mindestens eine erste und der mindestens eine zweite Gasanschluss Kalibriergas-, Nullgas- oder Messgasanschlüsse. So können alle für die Abgasmessung benötigten Gasanschüsse derart angeordnet werden, dass der Schaltschrank bauraumsparend ausgeführt ist und alle Gasanschlüsse und die Ventile leicht zugänglich sind.

In einer vorteilhaften Ausgestaltung sind der mindestens eine erste Gasanschluss und der mindestens eine zweite Gasanschluss mit jeweils einem an dem Schrankkörper befestigten Adapterelement befestigt, wobei das Adapterelement mindestens eine Einlassöffnung und mindestens eine Auslassöffnung aufweist, welche über das am Adapterelement angeordnete mindestens eine Ventil miteinander verbindbar sind. Dadurch werden der Gasanschluss und das dazugehörige Ventil einfach und bauraumsparend im Schrankkörper angeordnet. Außerdem werden der Montageaufwand und die Bauteileanzahl reduziert.

Vorzugsweise sind mehrere Adapterelemente zusammensteckbar und die in den Adapterelementen ausgebildeten Gaskanäle fluidisch miteinander verbindbar, wodurch eine Kalibiereinheit bereitgestellt werden kann, die abhängig von den benötigten Gasanschlüssen auf eine einfache Weise erweitert bzw. verkleinert sowie vormontiert werden kann.

In einer vorteilhaften Ausgestaltung sind mehrere Adapterelemente zu einer ersten Anschlussleiste und zu einer zweiten Anschlussleiste verbunden, wobei die erste Anschlussleiste eine Vielzahl an ersten Gasanschlüssen und die zweite Anschlussleiste eine Vielzahl an zweiten Gasanschlüssen aufweisen. Dadurch können mehrere Adapterelemente vor der Montage in den Schaltschrank zu einer Einheit vormontiert werden, wodurch die Montage in den Schaltschrank erleichtert und das Arbeiten von mehreren Personen am Schaltschrank ermöglicht wird.

Vorzugsweise sind die erste Anschlussleiste und die zweite Anschlussleiste jeweils über eine Befestigungsplatte am Schrankkörper befestigt, wodurch die Anschlussleisten komplett vormontiert im Schaltschrank befestigt werden können.

In einer bevorzugten Ausgestaltung ist im Schrankkörper mindestens ein dritter Gasanschluss angeordnet, welcher senkrecht zu dem mindestens einen ersten Gasanschluss und dem mindestens einen zweiten Gasanschluss ausgerichtet ist. Dadurch können drei Gasanschlüsse bauraumsparend im Schaltschrank angeordnet werden, indem beispielsweise zwei Gasanschlüsse an der Decke angeordnet sind und ein Gasanschluss an einer Seitenwand.

Vorzugsweise ist an den Befestigungselementen jeweils mindestens eine Platine angeordnet, die mit dem mindestens einen Ventil elektrisch verbunden ist. In einer besonders bevorzugten Ausgestaltung sind an den Befestigungselementen jeweils eine erste und eine zweite Platine angeordnet sind, die horizontal und vertikal versetzt zueinander angerordnet sind. Dadurch kann die Platine bauraumsparend im Schrankkörper in unmittelbarer Nähe zu den Ventilen angeordnet werden, so dass die Länge der elektrischen Leitungen reduziert wird.

Es wird somit ein Schaltschrank für Abgasmessanlagen geschaffen, der eine bauraumsparende Anordnung der Gasanschlüsse ermöglicht und zugleich die Gasanschlüsse sowie die zu den Gasanschlüssen dazugehörigen Ventile leicht zugänglich sind, wodurch die Wartung und die Montage des Schaltschranks erleichtert wird.

Ein Ausführungsbeispiel eines erfindungsgemäßen Schaltschranks für eine Abgasmessanlage ist in den Figuren dargestellt und wird nachfolgend beschrieben.

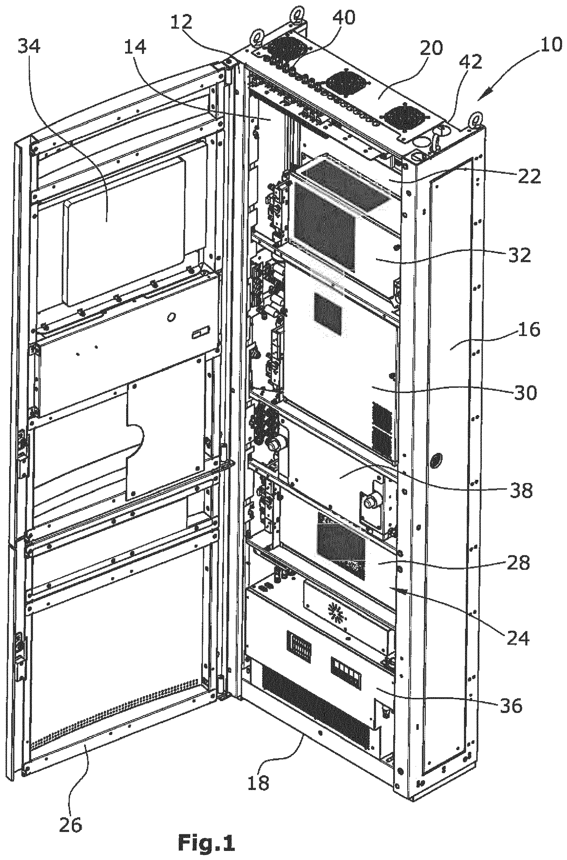

Figur 1 zeigt eine Frontansicht eines Schaltschranks für eine Abgasmessanlage mit einer erfindungsgemäßen Anordnung der Gasanschlüsse.
Figur 2 zeigt eine Seitenansicht des Schaltschranks aus Figur 1 mit einer erfindungsgemäßen Anordnung der Gasanschlüsse in geschnittener Darstellung.

Der Schaltschrank 10 für eine Abgasmessanlage besteht aus einem Schrankkörper 12 mit vier seitlich begrenzenden Wänden 14, 16, 18, 20, insbesondere einer ersten Seitenwand 14, einer gegenüberliegenden zweiten Seitenwand 16, einem Boden 18 und einer Decke 20 sowie einer Rückwand 22. Eine offene Frontseite 24 des Schrankkörpers 12 ist mittels einer Tür 26, welche schwenkbar an der ersten Seitenwand 14 befestigt ist, verschließbar.

In dem Schaltschrank 10 sind Messgeräte 28, 30, 32 zur Analyse von Messgasen angeordnet, welche mit an der Seitenwand 16 angeordneten und in den Figuren nicht sichtbaren Messgasanschlüssen verbunden sind und welche über eine in der Tür 26 angeordnete Bedieneinheit 34 ansteuerbar sind.

Im Schrankkörper 12 sind alle zur Analyse eines Messgases benötigten Komponenten angeordnet. Am Boden 16 des Schrankkörpers 12 ist eine Stromversorgungseinheit 36 angeordnet, über die die Messgeräte 28, 30, 32 und andere im Schränkkörper 12 angeordnete Komponenten mit Strom versorgt werden. Oberhalb der Stromversorgungseinheit 36 ist das erste Messgerät 28 angeordnet, welches ein Infrarotdetektoranalysegerät ist und zur Bestimmung von beispielsweise Kohlenmonoxid, Kohlendioxid oder Kohlenwasserstoffverbindungen eingesetzt wird. Das Messgas im Infrarotdetektoranalysegerät 28 wird durch einen über dem Infrarotdetektoranalysegerät 28 angeordneten Kühler 38 gekühlt und von Kondensat befreit.

Oberhalb des Kühlers 38 ist das zweite Messgerät 30 angeordnet, welches als Chemilumineszenzdetektoranalysegerät ausgeführt ist und über das die Menge des Stickoxids im Abgas gemessen wird.

Wiederum oberhalb des Chemilumineszenzdetektoranalysegeräts 30 ist als drittes Messgerät 32 ein Flammenionisationsdetektoranalysegerät zur Bestimmung von Kohlenwasserstoffen angeordnet.

An der Decke 20 des Schrankkörpers 12 sind zur Kalibrierung sowie zum Nullabgleich der Messgeräte 28, 30, 32 Gasanschlüsse 40, 42 angeordnet. Über die Gasanschlüsse 40, 42 werden Null- und Kalibriergase in den Schaltschrank 10 eingeleitet bzw. herausgleitet. Die Gasanschlüsse zum Einleiten von Messgasen sind in dieser Ausführung an der zweiten Seitenwand 16 angeordnet und in den Figuren nicht sichtbar. In anderen Ausführungen des Schaltschranks 10 könnten die Messgasanschlüsse in gleicher Weise wie die Gasanschlüsse 40, 42 ausgeführt und an der Decke 20 angeordnet sein.

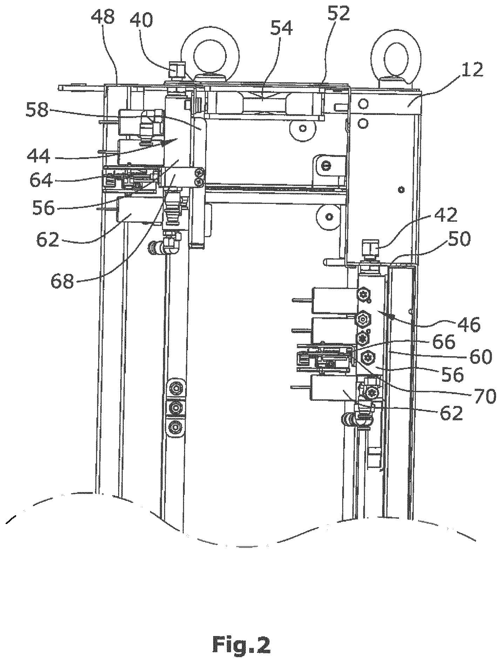

Figur 2 zeigt eine erfindungsgemäße Anordnung der Gasanschlüsse 40, 42 an der Decke 20 des Schrankkörpers 12. Dabei sind eine Vielzahl an ersten Gasanschlüssen 40 mit einer ersten Anschlussleiste 44 und eine Vielzahl an zweiten Gasanschlüssen 42 mit einer zweiten Anschlussleiste 46 verbunden, wobei die erste Anschlussleiste 44 horizontal und vertikal versetzt zur Anschlussleiste 46 im Schrankkörper 12 angeordnet ist. Die Decke 20 weist für eine derartige versetzte Anordnung der Anschlussleisten 44, 46 zwei Stufen 48, 50 auf. Die erste Stufe 48 schließt an die Frontseite 24 des Schrankkörpers 12 an und ist, von der mittleren Horizontalebene des Schrankkörpers 12 betrachtet, weiter außen angeordnet als die zweite Stufe 50. Die zweite Stufe 50 schließt an die Rückseite 22 des Schrankkörpers 12 an. So sind an der ersten Stufe 48 die erste Anschlussleiste 44 und an der zweiten Stufe 50 die zweite Anschlussleiste 46 angeordnet. Zwischen den beiden Stufen 48, 50 ist ein weiteres, leicht entfernbares Deckenelement 52 angeordnet, welches als Träger für einen Lüfter 54 dient und im eingebauten Zustand als Verlängerung der ersten Stufe 48 ausgeführt ist.

Die Anschlussleisten 44, 46 setzen sich jeweils aus mehreren zusammengesteckten Adapterelementen 56 zusammen und sind über jeweils ein Befestigungselement 58, 60 an der Rückwand 22 des Schrankkörpers 12 bzw. an der vertikalen erstreckenden Wand der ersten Stufe 48 befestigt. Jedes Adapterelement 56 weist eine zur Decke 20 gerichtete Öffnung mit einem Innengewinde auf, in die die als Einlassstutzen ausgeführten ersten und zweiten Gasanschlüsse 40, 42 eingeschraubt sind.

An die in das Adapterelement 56 eingeschraubten Gasanschlüsse 40, 42 schließt sich ein im Adapterelement 56 ausgebildeter und in den Figuren nicht dargestellter Gaskanal an, der sich ausgehend von den Gasanschlüssen 40, 42 bis zu einer ebenfalls nicht dargestellten Auslassöffnung erstreckt. Von der Auslassöffnung strömen die Null-, bzw. Kalibriergase zu den entsprechenden Messgeräten. Der Gasmassenstrom durch die Gasanschlüsse 40, 42 wird durch jeweils ein am Adapterelement 56 angeordnetes Ventil 62 freigegeben oder abgesperrt, gegebenenfalls auch geregelt.

Das Adapterelement 56 weist zusätzliche Gaskanäle auf, über die unterschiedliche Gasanschlüsse 40, 42 der jeweiligen Anschlussleiste 44, 46 miteinander verbunden sind.

Am unteren Ende der Anschlussleisten 44, 46 ist jeweils eine Platine 64, 66 angeordnet, an die die Ventile 62 elektrisch angeschlossen sind. Dabei sind die Platinen 64, 66 über jeweils eine Trägerplatte 68, 70 am Befestigungselement 58, 60 befestigt.

Bei der Wartung des Schaltschranks 10 müssen die Gasanschlüsse 40, 42 und die dazugehörigen Ventile 62 leicht zugänglich sein. Dabei soll der mit der Rückwand 22 an einer Gebäudewand stehende Schaltschrank 10 nicht verschoben werden. Die ersten Gasanschlüsse 40 der ersten Anschlussleiste 44 sind für das Wartungspersonal ausgehend von der Frontseite 24 leicht zugänglich, da die ersten Gasanschlüsse 40 direkt an dem der Frontseite 24 zugewandten Ende der Decke 20 angeordnet sind. Die zweiten Gasanschlüsse 42 sind ebenfalls von außen zugänglich. Um besser an die zweiten Gasanschlüsse 42 der zweiten Anschlussleiste 46 zu gelangen, ohne den Schaltschrank 10 zu verschieben, kann das Deckenelement 52 mit dem daran befestigten Lüfter 54 entfernt werden, wodurch die zweiten Gasanschlüsse 42 von oben oder von vorne erreichbar sind. Die Ventile 62 der beiden Anschlussleisten 44, 46, die im Schrankkörper 12 versetzt zueinander angeordnet sind, sowie die Verschraubung der Gasanschlüsse 42 sind bei geöffneter Tür 26 über die Frontseite 24 leicht zugänglich, da keine Leitungen vor der zweiten Anschlussleiste 46 entlang geführt werden müssen.

Somit wird ein Schaltschrank für Abgasmessanlagen geschaffen, der eine bauraumsparende Anordnung der Gasanschlüsse ermöglicht und zugleich die Gasanschlüsse sowie die zu den Gasanschlüssen dazugehörigen Ventile leicht zugänglich sind und dadurch die Wartung des Schaltschranks erleichtert.

Es sollte deutlich sein, dass der Schutzbereich des vorliegenden Hauptanspruchs nicht auf das beschriebene Ausführungsbeispiel begrenzt ist. Insbesondere kann der Schaltschrank auch mit anderen oder zusätzlichen Messgeräten bestückt werden. Auch können die Gasanschlüsse an einer der Seitenwände des Schrankkörpers in dieser zueinander versetzten Form angeordnet sein.

## Patentansprüche

1. Schaltschrank für Abgasmessanlagen mit
einem Schrankkörper (12), der sich aus vier seitlich begrenzenden Wänden (14, 16, 18, 20), von denen eine als Decke (20), eine als Boden (18) und zwei als Seitenwände (14, 16) dienen, sowie einer Rückwand (22) zusammensetzt und durch eine an der Frontseite (24) angeordnete Tür (26) verschließbar ist, wobei im Schrankkörper (12) Messgeräte (28, 30, 32) und Gasanschlüsse (40, 42) angeordnet sind, wobei sich die Gasanschlüsse (40, 42) durch die Wände des Schrankkörpers (12) erstrecken und jeweils mit mindestens einem innerhalb des Schrankkörpers (12) angeordneten Ventil (62) zusammenwirken,
wobei mindestens ein erster Gasanschluss (40) in einer ersten Horizontalebene und einer ersten Vertikalebene angeordnet ist und mindestens ein zweiter Gasanschluss (42) in einer zur ersten Horizontalebene versetzten zweiten Horizontalebene und einer zur ersten Vertikalebene versetzten zweiten Vertikalebene angeordnet ist, durch gekennzeichnet, dass die Decke (20) oder eine der Seitenwände (14, 16) stufenförmig mit einer ersten Stufe (48) und einer zweiten Stufe (50) ausgeführt sind, wobei an der ersten Stufe (48) der mindestens eine erste Gasanschluss (40) und an der zweiten Stufe (50) der mindestens eine zweite Gasanschluss (42) angeordnet sind.

2. Schaltschrank für Abgasmessanlagen nach Anspruch 1,
**dadurch gekennzeichnet, dass**
der mindestens eine erste Gasanschluss (40) näher zur Tür (26) angeordnet ist als der mindestens eine zweite Gasanschluss (42).

3. Schaltschrank für Abgasmessanlagen nach Anspruch 1 oder 2,
**dadurch gekennzeichnet, dass**
der mindestens eine erste Gasanschluss (40) und der mindestens eine zweite Gasanschluss (42) vertikal ausgerichtet sind und sich durch die Decke (20) des Schrankkörpers (12) erstrecken wobei der mindestens eine erste Gasanschluss (40) von der mittleren Horizontalebene des Schrankkörpers (12) weiter außen angeordnet ist als der mindestens eine zweite Gasanschluss (42).

4. Schaltschrank für Abgasmessanlagen nach Anspruch 1 oder 2,
**dadurch gekennzeichnet, dass**
der mindestens eine erste Gasanschluss (40) und der mindestens eine zweite Gasanschluss (42) horizontal ausgerichtet sind und sich durch eine Seitenwand (14;16) des Schrankkörpers (12) erstrecken wobei der mindestens eine erste Gasanschluss (40) von der mittleren Vertikalebene des Schrankkörpers (12) weiter außen angeordnet ist als der mindestens eine zweite Gasanschluss (42).

5. Schaltschrank für Abgasmessanlagen nach einem der vorangegangenen Ansprüche,
**dadurch gekennzeichnet, dass**
zwischen den beiden Stufen (48, 50) ein entfernbares Deckenelement (52) angeordnet ist.

6. Schaltschrank für Abgasmessanlagen nach einem der vorangegangenen Ansprüche,
**dadurch gekennzeichnet, dass**
der mindestens eine erste und der mindestens eine zweite Gasanschluss (40, 42) Kalibriergas-, Nullgas- oder
Messgasanschlüsse sind.

7. Schaltschrank für Abgasmessanlagen nach einem der vorangegangenen Ansprüche,
**dadurch gekennzeichnet, dass**
der mindestens eine erste Gasanschluss (40) und der mindestens eine zweite Gasanschluss (42) mit jeweils einem an dem Schrankkörper (12) befestigten Adapterelement (56) verbunden sind, wobei das Adapterelement (56) mindestens eine Einlassöffnung und mindestens eine Auslassöffnung aufweist, welche über das am Adapterelement (56) angeordnete mindestens eine Ventil (62) miteinander verbindbar sind.

8. Schaltschrank für Abgasmessanlagen nach Anspruch 7,
**dadurch gekennzeichnet, dass**
mehrere Adapterelemente (56) zusammensteckbar und die In den Adapterelementen (56) ausgebildeten Gaskanäle fluidisch miteinander verbindbar sind.

9. Schaltschrank für Abgasmessanlagen nach Anspruch 7 oder 8,
**dadurch gekennzeichnet, dass** mehrere Adapterelemente (56) zu einer ersten Anschlussleiste (44) und zu einer zweiten Anschlussleiste (46) verbunden sind, wobei die erste Anschlussleiste (44) eine Vielzahl an ersten Gasanschlüssen (40) und die zweite Anschlussleiste (46) eine Vielzahl an zweiten Gasanschlüssen (42) aufweisen.

10. Schaltschrank für Abgasmessanlagen nach Anspruch 9,
**dadurch gekennzeichnet, dass**
die erste Anschlussleiste (44) und die zweite Anschlussleiste (46) jeweils über ein Befestigungselement (58, 60) am Schrankkörper (12) befestigt sind.

11. Schaltschrank für Abgasmessanlagen nach Anspruch 10,
**dadurch gekennzeichnet, dass**
an den Befestigungselementen (58, 60) jeweils mindestens eine Platine (64, 66) angeordnet ist, die mit dem mindestens einen Ventil (62) elektrisch verbunden ist.

12. Schaltschrank für Abgasmessanlagen nach Anspruch 11,
**dadurch gekennzeichnet, dass**
an den Befestigungselementen (58, 60) jeweils eine erste und eine zweite Platine angeordnet sind, die horizontal und vertikal versetzt zueinander angeordnet sind.

13. Schaltschrank für Abgasmessanlagen nach einem der vorangegangenen Ansprüche,
**dadurch gekennzeichnet, dass**
im Schrankkörper (12) mindestens ein dritter Gasanschluss angeordnet ist, welcher senkrecht zu dem mindestens einen ersten Gasanschluss (40) und dem mindestens einen zweiten Gasanschluss (42) ausgerichtet ist.

## Claims

1. A switch cabinet for exhaust gas measurement installations comprising a cabinet body (12) which is composed of four laterally bounding walls (14, 16, 18, 20) of which one serves as a ceiling (20), one serves as a floor (18) and two serve as side walls (14, 16), as well as a rear wall (22), and can be closed by a door (26) arranged at the front side (24), wherein in said cabinet body (12) measuring devices (28, 30, 32) and gas connections (40, 42) are arranged, wherein said gas connections (40, 42) extend through the walls of said cabinet body (12) and respectively interact with at least one valve (62) arranged inside said cabinet body (12),
wherein at least one first gas connection (40) is arranged in a first horizontal plane and a first vertical plane, and at least one second gas connection (42) is arranged in a second horizontal plane offset to the first horizontal plane and a second vertical plane offset to the first vertical plane,
**characterized in that**
the ceiling (20) or one of the side walls (14, 16) are configured in a stepped manner with a first step (48) and a second step (50), wherein at said first step (48) the at least one first connection (40) is arranged and at said second step (50) the at least one second connection (42) is arranged.

2. The switch cabinet for exhaust gas measurement installations according to claim 1,
**characterized in that**
the at least one first gas connection (40) is arranged closer to the door (26) than the at least one second gas connection (42).

3. The switch cabinet for exhaust gas measurement installations according to claim 1 or 2,
**characterized in that**
the at least one first gas connection (40) and the at least one second gas connection (42) are vertically oriented and extend through the ceiling (20) of the switch cabinet (12), wherein said at least one first gas connection (40) is arranged further to the outside from the central horizontal plane of the cabinet body (12) than said at least one second gas connection (42).

4. The switch cabinet for exhaust gas measurement installations according to claim 1 or 2,
**characterized in that**
the at least one first gas connection (40) and the at least one second gas connection (42) are horizontally oriented and extend through a side wall (14; 16) of the cabinet body (12), wherein said at least one first gas connection (40) is arranged further to the outside from the central vertical plane of said cabinet body (12) than said at least one second gas connection (42).

5. The switch cabinet for exhaust gas measurement installations according to any one of the preceding claims,
**characterized in that**,
between the two steps (48, 50) a removable ceiling element (52) is arranged.

6. The switch cabinet for exhaust gas measurement installations according to any one of the preceding claims,
**characterized in that**
the at least one first and the at least one second gas connection (40, 42) are calibrating gas, zero gas or measuring gas connections.

7. The switch cabinet for exhaust gas measurement installations according to any one of the preceding claims,
**characterized in that**
the at least one first gas connection (40) and the at least one second gas connection (42) are connected to a respective adapter element (56) fastened at the cabinet body (12), wherein said adapter element (56) comprises at least one inlet opening and at least one outlet opening which are connectable to each other via the at least one valve (62) arranged at said adapter element (56).

8. The switch cabinet for exhaust gas measurement installations according to claim 7,
**characterized in that**
several adapter elements (56) are adapted to be assembled and the gas ducts formed in the adapter elements (56) are fluidically connectable to each other.

9. The switch cabinet for exhaust gas measurement installations according to claim 7 or 8,
**characterized in that**
several adapter elements (56) are connected to a first connection strip (44) and to a second connection strip (46), wherein said first connection strip (44) comprises a plurality of first gas connections (40) and said second connection strip (46) comprises a plurality of second gas connections (42).

10. The switch cabinet for exhaust gas measurement installations according to claim 9,
**characterized in that**
the first connection strip (44) and the second connection strip (46) are respectively fastened to the cabinet body (12) via a fastening element (58, 60).

11. The switch cabinet for exhaust gas measurement installations according to claim 10,
**characterized in that**,
at the fastening elements (58, 60), respectively at least one circuit board (64, 66) is arranged which is electrically connected to the at least one valve (62).

12. The switch cabinet for exhaust gas measurement installations according to claim 11,
**characterized in that**,
at the fastening elements (58, 60) a first and a second circuit board are respectively arranged such that they are horizontally and vertically offset to each other.

13. The switch cabinet for exhaust gas measurement installations according to any one of the preceding claims,
**characterized in that**,
in the cabinet body (12) at least one third gas connection is arranged which is vertically oriented to the at least one first gas connection (40) and the at least one second gas connection (42).

## Revendications

1. Armoire de commande pour systèmes de mesure de gaz d'échappement comprenant
un corps d'armoire (12) formé de quatre parois (14, 16, 18, 20) délimitant latéralement, dont une sert comme plafond (20), une sert comme fond (18) et deux servent comme parois latérales (14, 16), ainsi qu'une paroi arrière (22) et pouvant être fermé par une porte (26) disposée à la face avant (24), des dispositifs de mesure (28, 30, 32) et des raccords de gaz (40, 42) étant disposés dans le corps d'armoire (12), les connexions de gaz (40, 42) s'étendant à travers les parois du corps d'armoire (12) et coopérant chacune avec au moins une soupape (62) disposée à l'intérieur du corps d'armoire (12),
au moins une première connexion de gaz (40) étant disposée en un premier plan horizontal et un premier plan vertical et au moins une deuxième connexion de gaz (42) étant disposée dans un deuxième plan horizontal décalé par rapport au premier plan horizontal et un deuxième plan vertical décalé par rapport au premier plan vertical,
**caractérisé en ce que**
le plafond (20) ou l'une des parois latérales (14, 16) sont conçus en gradins avec un premier niveau (48) et un deuxième niveau (50), l'au moins une première connexion (40) étant disposée sur le premier niveau (48) et l'au moins une seconde connexion (42) étant disposée sur le deuxième niveau (50).

2. Armoire de commande pour systèmes de mesure de gaz d'échappement selon la revendication 1, **caractérisée en ce que** l'au moins une première connexion de gaz (40) est disposée plus près de la porte (26) que l'au moins une deuxième connexion de gaz (42).

3. Armoire de commande pour systèmes de mesure de gaz d'échappement selon la revendication 1 ou 2, **caractérisée en ce que** l'au moins une première connexion de gaz (40) et l'au moins une seconde connexion de gaz (42) sont alignées verticalement et s'étendent à travers le plafond (20) du corps d'armoire (12), ladite au moins une première connexion de gaz (40) étant disposée plus à l'extérieur du plan horizontal central du corps d'armoire (12) que l'au moins une deuxième connexion de gaz (42).

4. Armoire de commande pour systèmes de mesure de gaz d'échappement selon la revendication 1 ou 2, **caractérisée en ce que** l'au moins une première connexion de gaz (40) et l'au moins une deuxième connexion de gaz (42) sont alignées horizontalement et s'étendent à travers une paroi latérale (14; 16) du corps d'armoire (12), l'au moins une première connexion de gaz (40) étant disposée plus à l'extérieur du plan vertical central du corps d'armoire (12) que l'au moins une deuxième connexion de gaz (42).

5. Armoire de commande pour systèmes de mesure de gaz d'échappement selon l'une quelconque des revendications précédentes, **caractérisée en ce qu'**un élément de plafond amovible (52) est disposé entre les deux niveaux (48, 50).

6. Armoire de commande pour systèmes de mesure de gaz d'échappement selon l'une quelconque des revendications précédentes, **caractérisée en ce que** l'au moins une première et l'au moins une deuxième connexion de gaz (40, 42) sont des connexions de gaz d'étalonnage, de gaz zéro ou de gaz de mesure.

7. Armoire de commande pour systèmes de mesure de gaz d'échappement selon l'une quelconque des revendications précédentes, **caractérisée en ce que** l'au moins une première connexion de gaz (40) et l'au moins une deuxième connexion de gaz (42) sont chacune raccordées à un élément adaptateur (56) fixé au corps d'armoire (12), l'élément adaptateur (56) présentant au moins une ouverture d'entrée et au moins une ouverture de sortie, qui peuvent être reliées l'une à l'autre via l'au moins une soupape (62) disposée sur l'élément adaptateur (56).

8. Armoire de commande pour systèmes de mesure de gaz d'échappement selon la revendication 7, **caractérisée en ce que** plusieurs éléments adaptateurs (56) peuvent être enfichés l'un dans l'autre et les canaux de gaz formés dans les éléments adaptateurs (56) peuvent être reliés fluidiquement les uns aux autres.

9. Armoire de commande pour systèmes de mesure de gaz d'échappement selon la revendication 7 ou 8, **caractérisée en ce que** plusieurs éléments adaptateurs (56) sont reliés pour former un premier bloc de connexion (44) et un deuxième bloc de connexion (46), le premier bloc de connexion (44) comprenant plusieurs premières connexions de gaz (40) et le deuxième bloc de connexion (46) comprenant plusieurs deuxième connexions de gaz (42).

10. Armoire de commande pour systèmes de mesure de gaz d'échappement selon la revendication 9, **caractérisée en ce que** le premier bloc de connexion (44) et le deuxième bloc de connexion (46) sont chacun fixés au corps d'armoire (12) au moyen d'un élément de fixation (58, 60).

11. Armoire de commande pour systèmes de mesure de gaz d'échappement selon la revendication 10, **caractérisée en ce qu'**au moins une carte de circuit imprimé (64, 66) est disposée, respectivement, sur les éléments de fixation (58, 60), la carte étant connectée électriquement à l'au moins une soupape (62).

12. Armoire de commande pour systèmes de mesure de gaz d'échappement selon la revendication 11, **caractérisée en ce qu'**une première et une deuxième carte de circuits imprimés sont disposées sur les éléments de fixation (58, 60), qui sont disposés horizontalement et verticalement décalés l'un par rapport à l'autre.

13. Armoire de commande pour systèmes de mesure de gaz d'échappement selon l'une des revendications précédentes, **caractérisée en ce qu'**au moins une troisième connexion de gaz est disposée dans le corps d'armoire (12), qui est orientée perpendiculairement à l'au moins une première connexion de gaz (40) et l'au moins une deuxième connexion de gaz (42).
